# EUROPEAN PATENT APPLICATION

(11) **EP 1 719 590 A1**
(43) Date of publication of application: **08.11.2006**
(21) Application number: 06116524.7
(22) Date of filing: 13.05.2004
(51) Int. Cl.: B26B 21/44

(54) **Composition for shaving aid material and shaving aid cartridge for shaving aid material**

(30) Priority: 16.05.2003 US 471037 P
(62) Divisional of application: 04785535.8
(71) Applicant: Eveready Battery Company, Inc., St Louis, Missouri 63141 (US)
(72) Inventor: Sandor, James A., Trumbull, CT 06611 (US); Defeo, Martha, Milford, CT 06460 (US); Orloff, Glennis J., Woodbridge, CT 06525 (US); Mehta, Rooma M., Guilford, CT 06437 (US)
(74) Representative: Hilleringmann, Jochen

(57) **Abstract**

A shaving aid cartridge (10) includes a base (14) adapted to he coupled to a shaving implement (11) and a body (12) coupled to the base. The body is formed at least in part from a shaving aid preparation. The shaving aid cartridge defines a separation force at an interface between the body and the base of about 8 to about 30 pounds. Another embodiment of the shaving aid cartridge defines an energy to break of about 0.2 to about 2.0 foot pounds. Another embodiment of the shaving aid cartridge includes a body that defines an impact resistance of about 0.05 to about 0.40 inches. Another embodiment of a shaving aid cartridge includes a body that defines a wear rate of about 0.002 to about 0.040 millimeters per cycle. Yet another embodiment of a shaving aid includes a body that defines a hardness of about 0.2 to about 44.5 Newtons. In still another embodiment, the shaving aid cartridge includes a base adapted to be coupled to a shaving implement and a body coupled to the base. The body is formed at least in part from a shaving aid preparation that comprises a composition sapomified or neutralized from a vegetable oil, tallow, a surface-eroding polymer, or a combination of at least two of the foregoing materials.

## Description

### Field of the Present Invention

The present invention relates to shaving aid cartridges, and, more particularly, to shaving aid compositions for use with shaving aid cartridges.

### Background of the Present Invention

A safety razor typically includes a disposable razor cartridge mounted in a reusable handle, or a handle and a cartridge combined into a unitary disposable unit. Most razor cartridges include a frame, at least one razor blade, and a strip of shaving aid material attached to the frame to enhance the shaving process The shaving aid material facilitates the movement of the razor blade over the skin and/or aids in the removal of hair from the skin. Shaving aid materials include, but are not limited to, lubricating agents, drag-reducing agents, depilatory agents, cleaning agents, medicinal agents, and the like.

Most safety razors are designed for use with a shaving preparation. The shaving preparation (e.g., shaving cream, gel, or the like) is applied to the skin to condition and soften the skin and further assists in the softening of the hair to be shaved. Generally, the preparation remains on the skin until it is removed during the shaving process or in the rinsing of the skin upon completion of the shaving process.

While conventionally packaged shaving preparations are advantageous in preparing a hirsute surface for a shaving operation, there are drawbacks to their use. For example, when one shaves in the shower, it is difficult to employ a conventional shaving preparation because the preparation tends to wash off the surface. Moreover, where large areas such as a person's legs are to be shaved, significant amounts of shaving aid preparation may be needed to adequately coat the areas. Application of the shaving aid preparation takes time and is potentially a two-handed process.

Another problem sometimes encountered is that the shaving aid preparation is typically packaged separate from a razor. Accordingly, one must remember to bring the shaving aid preparation into the shower with them. Furthermore, when one is travelling, the shaving aid preparation must be packed, thereby taking up space and adding weight to one's luggage.

Based on the foregoing, it is the general object of the present invention to provide a shaving aid cartridge that overcomes or improves upon the prior art.

### Summary of the Present Invention

The present invention is directed in one aspect to a shaving aid cartridge that includes a base adapted to be coupled to a shaving implement. A body is coupled to the base and is formed at least in part from a shaving aid preparation. As used herein, it should be understood that the term "shaving aid preparation" includes combinations of shaving aid preparations and shaving aid materials. A tensile force required to cause the body to separate from the base at an interface thereof is defined herein as the "separation force" and is in the range of about 8 to about 30 pounds. The manner by which this force is exerted and measured is described below in the section entitled "Detailed Description of the Preferred Embodiments."

The separation force is one of several different characteristics of the present invention that can be used to characterize the shaving aid cartridge. Another characteristic that, by itself, can be used to distinguish the shaving aid cartridge of the present invention is referred to as the "energy-to-break." The energy-to-break is a measure of the dynamic force required to break at least a portion of the body away from the base. The body in the present invention exhibits an energy-to-break of about 0.2 to 2.0 foot pounds. The manner in which this is measured is described below in the section labeled "Detailed Description of the Preferred Embodiments."

Still another mechanical property which, separate from the above-described mechanical properties, can be used to characterize the present invention is impact resistance. The impact resistance is a measure of the resistance of the material to deform. Preferably, an acceptable level of deformation, and thereby impact resistance should be in the range of about 0.05 to about 0.40 inches. The manner in which this is measured is also described below in the section labeled "Detailed Description of the Preferred Embodiments."

The present invention also resides in a shaving aid preparation that exhibits a wear rate of about 0.002 to about 0.040 millimeters per cycle. The wear rate is defined by the resistance of the shaving aid to an erosive condition. The manner in which this is measured and what is meant by the term "cycle" is also described below in the section labeled "Detailed Description of the Preferred Embodiments."

Preferably, the body is formed at least in part from a shaving aid preparation that comprises a surface eroding polymer, a synthetic detergent, a super-fatted material, glycerin, a composition saponified or neutralized from a vegetable oil or a tallow, or a combination of at least two of the foregoing materials.

One advantage of the present invention is that the shaving aid preparation is applied as needed as a shaving operation progresses. By applying the shaving aid preparation during the shaving process (e.g., in a shower), the potential for it being prematurely washed away is minimized Because the shaving aid preparation is applied in the very least just prior to the blades, maximum lubricity and thereby shaving comfort is achieved.

Another advantage of the present invention is that the shaving aid preparation is compatible with use in the shower, In particular, the shaving aid preparation is configured to withstand conditions of the shower without being washed away.

Still another advantage of the shaving aid preparation employed in the present invention is that it exhibits mechanical characteristics that allow the shaving aid preparation to maintain its structural integrity over prolonged periods of time and in a sometimes severe environment

### Brief Description Of The Drawings

FIG 1 is a side elevation view of a razor assembly including a shaving aid cartridge in accordance with the present invention.
FIG. 2 is a perspective view of a shaving aid cartridge in which the shaving aid defines an aperture into which a razor cartridge is positioned.
FIG. 3 is a perspective view of a shaving aid cartridge in which shaving aid preparation in the form of discrete elements is positioned adjacent to, but spaced away from, the leading and trailing surfaces of a razor cartridge.
FIG. 4 is a perspective view of a base shown in FIG 2.
FIG. 5, FIG. 6, and FIG. 7 arc perspective views of bases for shaving aid cartridges.

### Detailed Description of the Preferred Embodiments of the Present Invention

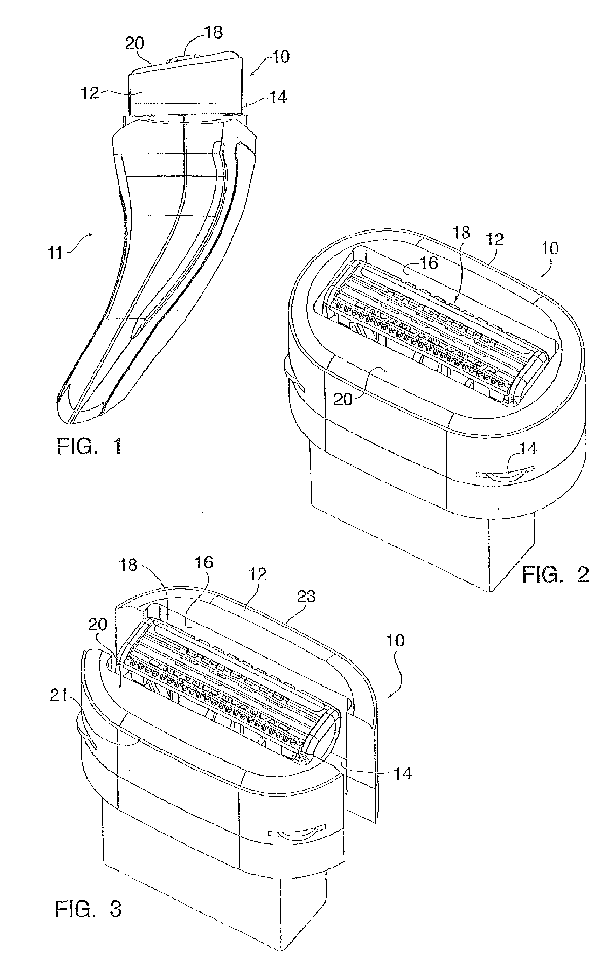

Referring to FIGS. 1-3, a shaving aid cartridge 10 for use with a razor assembly generally designated by the reference numeral 11 includes a shaving aid body 12 attached to a base 14 The shaving aid body 12 includes an aperture 16 into which a razor cartridge 18, wholly separate from the shaving aid body, is positioned. The shaving aid body 12 includes a contact surface 20 that, during a shaving operation, engages a hirsute surface, In the illustrated embodiment, the shaving aid body 12 surrounds and is spaced away from the razor cartridge 18 and is substantially oval-shaped However, the present invention is not limited in this regard as other shapes such as, for example, circular shapes, rectangular shapes, or virtually any other geometric configuration can be substituted without departing from the broader aspects of the present invention. As shown in FIG. 3, the shaving aid body 12 comprises a forward portion 21 spaced away from but positioned relative to a leading edge of the razor cartridge 18 and an aft portion 23 spaced away from but positioned relative to a trailing edge of the razor cartridge. The forward portion 21 and the aft portion 21 may be rounded or otherwise configured.

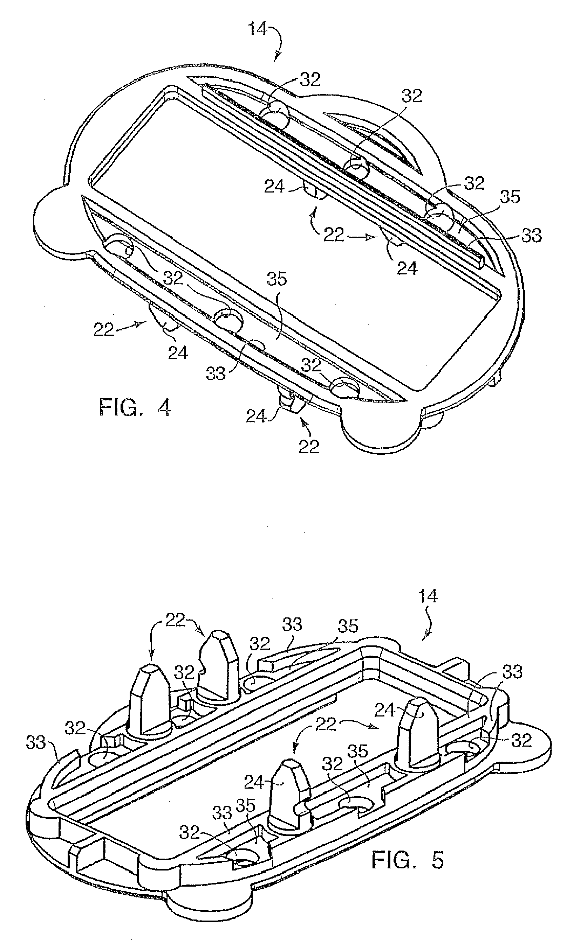

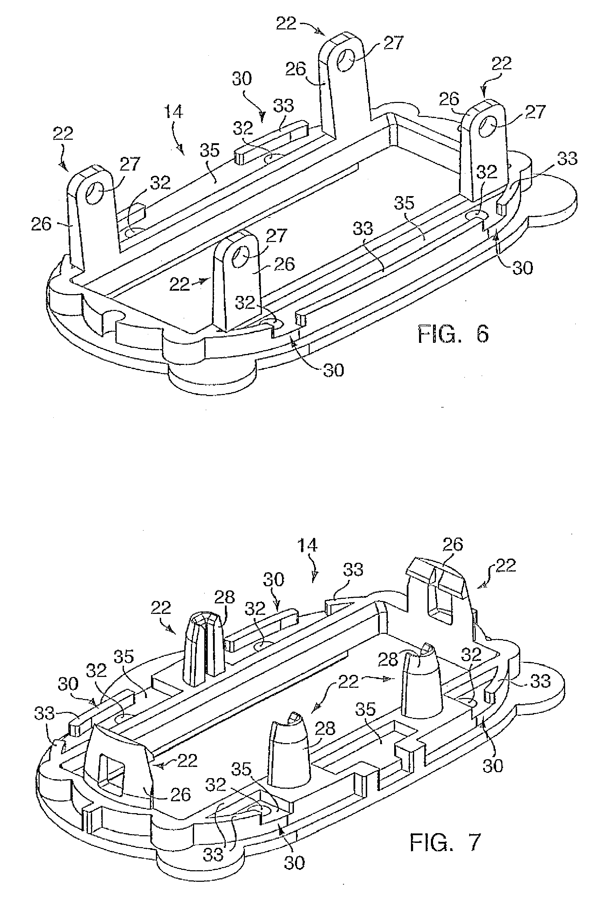

As shown in FIGS. 4-7, the base 14 includes a rigid structure having features 22 that facilitate the attachment of the shaving aid cartridge 10 to the razor assembly 11 (FIG. 1). The features 22 include tabs, flanges, or the like that enable the shaving aid cartridge to be mechanically attached to the razor assembly. In particular, FIGS. 4 and 5 show the base 14 as having a plurality of tabs 24. Figure 6 shows the base 14 having a plurality of flanges 26 having apertures 27. Figure 7 shows the base 14 having a plurality of grooved flanges 28. The type and configuration of the features 22 operable to attach the shaving aid cartridge 10 to the razor assembly can be varied to adapt to the application at hand.

Still referring to FIGS 4-7, the base 14 further includes features 30 operable to aid in attaching the shaving aid body 12 to the base 14. The features 30 for attaching the shaving aid body 12 to the base 14 may include, for example, one or more apertures 32, protrusions 33, and/or voids 35 that the shaving aid material flows within and around during a forming operation. The apertures 32, protrusions 33 and/or voids 35 are preferably configured and positioned such that during formation of the shaving aid cartridge 10, the shaving aid body 12 and the base 14 are fixed both mechanically and adhesively to each other by solidified shaving aid material disposed within and around the apertures 32, protrusions 33, and/or voids 35 within the base 14. The base 14 preferably is formed from a thermoplastic material by a method such as injection molding. However, the present invention is not limited in this regard as other materials such as, but not limited to, metals, thermosetting materials, or composite materials can also be employed.

Referring back to FIGS 1-3, the preferred embodiment of the shaving aid body 12 comprises an erodable shaving aid preparation which is lubricious and can have some skin cleansing and/or conditioning properties. The basic structure of the shaving aid body 12 may be that of a vegetable oil or tallow, saponified or neutralized to form the base, a combination of base and synthetic surfactants, non- soap based synthetic detergents, combinations of the foregoing compositions, or the like. Super-fatted materials containing percentages (e.g., greater than about 25 weight percent) of coconut acid or other fatty acids may also be used. The term "crodable" is hereinafter intended to refer to the ability of the shaving aid material to be worn away by the flow of water or as the result of abrasion.

In one embodiment of the shaving aid body 12, the shaving aid preparation is a composition having a base comprising a vegetable oil or a tallow or the like, or a combination of the foregoing materials, which is saponified or neutralized The saponification or neutralization of the vegetable oil or tallow results in the production of glycerol and salts of fatty acids to form the base. The composition preferably comprises about 75 weight percent (wt. %) to about 100 wt. % saponified or neutralized base, which may be opaquc, translucent, or transparent. Exemplary salts of fatty acids that may be produced include sodium carboxylate salts having up to about 22 carbon atoms.

In another embodiment of the shaving body 12, the shaving aid preparation is a composition having a base comprising a synthetic detergent, glycerin, a surface-eroding polymer, or a combination comprising at least two of the foregoing materials. The composition preferably comprises about 75 to about 100 wt. % base, which may be opaque, translucent, or transparent.

In still another embodiment of the shaving body 12, the shaving aid preparation is a composition having a base comprising a synthetic detergent, glycerin, a surface-eroding polymer, or a combination of at least two of the foregoing and a vegetable and/or tallow product resulting from saponification-or neutralization. The composition preferably comprises about 75 to about 100 wt. % base, which may be opaque, translucent, or transparent.

In any embodiment, the balance of the composition may include various skin care ingredients, binding agents, absorbents, and other additives. The skin care ingredients that may be added to the base to enhance the composition include, but are not limited to, surfactants, skin benefiting agents (e.g., emollients, lubricants, humectants, moisturizing agents, conditioners, and the like), foaming agents, hair growth inhibitors, botanical extracts, antioxidants, antimicrobials, anti- inflammatory agents, astringents, anti-irritants, depilatory agents, medicinal agents, and exfoliating agents (e.g., loofa, seaweed, oatmcal, pumice, apricot seed, and the like). Exemplary embodiments of skin care ingredients include, but are not limited to, sodium or potassium salts (e.g., lactylates, chlorides, sulfonates, and the like), vitamins and vitamin complexes, cocoates, metal oxides, oils (e.g., cocoa butter), and aloe barbadensis. The absorbents are preferably clays or clay-based compositions, chalks, talcs, silicas, or the like. Clays that may be added include bentonite, kaolin, combinations of the foregoing clays, and the like.

In one embodiment of a composition, a vegetable oil is saponified to produce a base. The composition may further comprise at least one other surfactant such as, for example, sodium isostearoyl lactylate, ammonium isostearate, DEA-myristate, alkyl glyceryl sulfonate, laureth-16 combinations of at least two of the foregoing materials, or the like, in an amount up to about 9 wt. %, preferably up to 7.5 wt. % and more preferably up to about 6 wt. % ; at least one skin-conditioning agent such as, for example, aloe barbadensis, dimethicone, allantoin, sucrose cocoate, oleyl lanolate, thiourea, cocoa butter, tocopheryl acetate, PPG-33, undeceth-3, honey, algae, combinations of at least two of the foregoing materials, or the like, in an amount up to about 14 wt. %, preferably up to 8 wt. %, and more preferably up to about 2 wt. % ; at least one absorbent such as, for example, kaolin, chalk, tale, hydrated silica, wood powder, sodium chloride, cyclodextrin, combinations of at least two of the foregoing materials, or the like, in an amount up to about 9 wt. %, preferably up to 5 wt. %, and more preferably up to about 3 wt. %. While various surfactants, skin conditioning agents, absorbents, coloring agents, and fragrances have been shown and described, the present invention is not limited in this regard as other materials known to those skilled in the pertinent art to which the present invention pertains may be substituted and/or replaced.

One embodiment of the above-described composition may be suited for users having skin that is not sensitive to the dyes or pigments used as coloring agents. Thus, the composition may further comprise at least one coloring agent such as, for example, titanium dioxide, manganese violet, zinc oxide, an Ultramarine (e.g., Ultramarine Blue 4), Orange 4, Green 3, combinations of at least two of the foregoing materials, or the like, in an amount up to about 6 wt. %, preferably up to 4 wt. %, and more preferably up to about 1 wt. %. However, the present invention is not limited in this regard as coloring agents other than those listed previously may be substituted without departing form the broader aspects of the present invention.

Additionally, fragrances may be added to the composition Fragrances are odorants used to impart desirable smells to the composition and may further mask the less desirable odors of other components of the composition. In an embodiment that may be suited for users having skin that is not sensitive to the fragrances, the composition may further comprise at least one fragrance ingredient in the amount up to about 2 wt. %, preferably up to 1.5 wt, % and more preferably up to about 1 wt. %.

In any embodiment of the composition, the mechanical properties thereof enable the shaving aid body to withstand normal use while experiencing a corresponding degree of wear and strength. The mechanical properties of the composition also preferably allow the shaving aid body 12 to be able to withstand some degree of abuse without experiencing significant damage to the shaving aid body. Thus, the mechanical properties of the composition may be defined at least in part by the hardness of the composition, the tensile strength required for the separation of the shaving aid body 12 from the base 14, the ability of the composition to resist impact, and the ability of the composition to resist wear.

The hardness of the composition (the resistance of the composition to deform) may be determined by a Durometer hardness test method (e.g., ASTM D 2240 Standard Test Method for Rubber Property-Durometer Hardness). In one exemplary test method, a sample of the composition is mounted or placed on a suitable testing apparatus in which a test head engages a surface of the composition sample. A hardness value is obtained based on a calculation derived from various parameters, e.g., pressure exerted by or on the test head, movement of the test head, etc. A suitable testing apparatus is the Shore Instruments Automatic Operating Stand Model 902. The composition from which the shaving aid body 12 is fabricated has a hardness of about 0.2 to about 44.5 Newtons (N), preferably about 0.5 to about 22.5 N, and more preferably about 4.0 to about 10.0 N.

The tensile strength required for the separation of the shaving aid body 12 from the base 14 may be determined by a tensile force separation test. In such a test, the shaving aid cartridge 10 is mounted on a suitable apparatus in which the base 14 is securely retained. At least one knife attached to a carrier is inserted into the shaving aid body 12. The carrier is movable relative to the fixed position of the base 14. Preferably, the carrier is translatable in a direction that is substantially orthogonal to a plane defined by an interface of the shaving aid body 12 and the base 14, Upon movement of the carrier, the force required for the separation of the shaving aid body 12 from the base 14 is measured. A suitable tensile strength testing apparatus is the Chatillon TCM 201 Tensile Test Machine fitted with the Chatillon DFM 100 CE Force Gauge and modified to have custom holding fixtures.
The force gauge measures the amount of force required to separate the shaving aid body 12 from the base 14. The force required for the separation of the shaving aid body 12 from the base 14 is about 8 to about 30 pounds (lbs.), preferably about 8 to about 26 lbs., and more preferably about 10 to about 22 lbs.

The ability of the shaving aid body 12 to retain its structural integrity when dropped is determined by measuring the energy required to break the composition, referred to by those of skill in the pertinent art to which the invention pertains as "energy-to-break." An embodiment of an apparatus utilized to determine the energy to break the composition is a swing arm testing device. A swing arm testing device employs a pendulum having a hammer disposed at a non-fulcrum end of the pendulum. Because the mass of the hammer and the pendulum are known, and because the height from which the hammer is swung is measured, the force exerted by the hammer at a preselected impact point at which the shaving aid body 12 is fixedly mounted can be calculated. The impact point is taken to be the point determined to be most vulnerable to damage, i.e., the portions of the shaving aid body that connects the front portion to the aft portion. The thickness of the wall of the shaving aid body 12 in the direction of the impact force is about 0.2 inches (in.) Thus, the energy required to break the shaving aid body 12 into at least two portions is about 0.2 to about 2.0 foot pounds (ft. lbs.), preferably about 0.3 to about 1.0 ft. lbs., and more preferably about 0.4 to about 0.53 ft. lbs. A suitable swing arm testing device useful in performing the above-desired test is a Satec Systems, Inc. BLI Impact Tester.

Also determinable from the swing arm testing device is the impact resistance of the composition. In tests in which the composition does not break, an analysis of a resulting deformation caused by the impact of the hammer can be made. The resultant value (e.g., the distance that the surface of the composition is indented) is measured in linear units. The impact resistance (at 0.53 ft. 1bs.) of the composition is about 0.05 to about 0.40 in. , preferably about 0.08 to about 0.30 in. , and more preferably about 0.10 to about 0.20 in

The ability of the composition to resist wear is an indication of the usable life of the shaving aid cartridge 10. Wear resistance is measured by maintaining a flow of water over a textured surface and between this textured surface and the shaving aid body 12 in a water bath at a pre-selected temperature and abrading the shaving aid body 12 to simulate a shaving process. The flow of water through the water hath is about 0.08 gallons per minute (gpm), and the temperature of the water is about 38 degrees C to about 42 degrees C Abrading the shaving aid body 12 is effected by an apparatus that provides for the engagement of the shaving aid body 12 by a textured surface and movement of the shaving aid body 12 across the textured surface in an oscillating motion. The textured surface is preferably a stainless steel pad having a surface finish number of 32 microinches (Ra) (about 150 grit). Each continuous movement (forward and back) of the shaving aid body 12 over the textured surface is a cycle. One complete cycle is a movement of about six inches in each direction (forward and back). A load of 300 grams (g) is maintained on the shaving aid body 12 using weights. The wear rate of the shaving aid body 12 is about 0.002 to about 0 040 millimeters per cycle (mm/cycle), preferably about 0.004 to about 0.030 mm/cycle, and more preferably about 0.008 to about 0.016 mm/cycle

One exemplary method of manufacturing the shaving aid preparation comprises synthesis by saponification or neutralization. In this exemplary method, a crutching process is utilized to blend various ingredients as a secondary phase for addition to the saponification or neutralization product. Subsequent to the crutching process, a drying process is employed to remove water The resulting dried material is then pelletized and subjected to an amalgamating process. In the amalgamating process, additional ingredients may be added to produce a final pelletized composition.

### Example

A shaving aid preparation for a shaving aid cartridge was prepared with a base saponified from vegetable oil. The base was blended with surfactants, skin conditioning agents, absorbents, colorants, and fragrances. The composition of the resulting shaving aid preparation is shown below:

| Component | Wt.% |
|---|---|
| Base | 82.5 |
| Surfactants | 7.0 |
| Skin conditioning agents | 5.0 |
| Absorbents | 2.0 |
| Colorants | 2.0 |
| Fragrances | 1.5 |

Although this invention has been shown and described with respect to the detailed embodiments thereof, it will be understood by those of skill in the art that various changes may be made and equivalents may be substituted for elements thereof without departing from the scope of the invention. In addition, modifications may be made to adapt a particular situation or material to the teachings of the invention without departing from the essential scope thereof. Therefore, it is intended that the invention not be limited to the particular embodiments disclosed in the above detailed description, but that the invention will include all embodiments falling within the scope of the appended claims. For example, although the above description is directed to a shaving aid cartridge, it should be understood by those of skill in the art that aspects of the above-described apparatus may be applicable to articles other than shaving aid cartridges.

## Claims

1. A shaving aid cartridge (10), **characterized by**:
- a base (14) adapted to be coupled to a razor assembly (11); and
- a body (12) coupled to said base (14), said body (12) being formed at least in part from a shaving aid preparation; and wherein
- said shaving aid preparation comprises a synthetic detergent, glycerin, a surface- eroding polymer, a composition saponified or neutralized from a vegetable oil or a tallow, or a combination of at least two of the foregoing materials.

2. The shaving aid cartridge (10) of claim 1, wherein said shaving aid preparation further comprises a surfactant, a skin care ingredient, an absorbent, a binding agent, an exfoliating agent, an antimicrobial agent, an emollient, a humectant, a moisturizing agent, a skin conditioning agent, a hair growth inhibitor, an essential oil, a botanical extract, an antioxidant, an anti-inflammatory agent, an astringent, an anti-irritant, a vitamin, a depilatory agent, a medicinal agent, or a combination of at least two of the foregoing materials.

3. The shaving aid cartridge (10) of claim 1 or 2, wherein said shaving aid preparation further comprises a fragrance.

4. The shaving aid cartridge (10) of any one of claims 1 to 3, wherein said shaving aid preparation further comprises a coloring agent.

5. The shaving aid cartridge (10) of any one of claims 1 to 4, wherein said surfactant is sodium isostearoyl lactylate, ammonium isostearate, DEA-myristate, alkyl glyceryl sulfonate, laureth-16, or a combination of at least two of the foregoing materials.

6. The shaving aid cartridge (10) of claim 5, wherein said surfactant is present in an amount up to about 9 wt. % or in an amount up to about 7.5 wt. % or in an amount up to about 6 wt. %.

7. The shaving aid cartridge (10) of any one of claims 1 to 6, wherein said synthetic detergent, said glycerin, said surface-eroding polymer, said composition saponified or neutralized from vegetable oil or tallow, or said combination of at least two of the foregoing materials is present in an amount of about 75 wt. % to about 100 wt. %.
